# EUROPÄISCHE PATENTSCHRIFT

(11) **EP 0 369 364 B1**
(45) Veröffentlichungstag und Bekanntmachung des Hinweises auf die Patenterteilung: **28.09.1994**
(21) Anmeldenummer: 89120949.6
(22) Anmeldetag: 11.11.1989
(51) Int. Cl.: B01J 29/28, C10G 3/00, C07C 1/20

(54) **Katalysator auf der Basis von kristallinem Alumosilikat**
Catalyst based on a crystalline aluminosilicate
Catalyseur à base d'un aluminosilicate cristallin

(30) Priorität: 15.11.1988 DE 3838710
(43) Veröffentlichungstag der Anmeldung: 23.05.1990
(73) Patentinhaber: SÜD-CHEMIE AG, D-80333 München (DE)
(72) Erfinder: Burgfels, Götz, Dr. Dipl.-Chem., D-8210 Feilenbach-Au (DE); Kochloefl, Karl, Dr. Dipl.-Ing., D-8206 Bruckmühl 2 (DE); Ladebeck, Jürgen, Dr. Dipl.-Chem., D-8202 Bad Aibling (DE); Schmidt, Friedrich, Dr. Dipl.-Chem., D-8200 Rosenheim (DE); Schneider, Michael, Dr. Dipl.-Chem., D-8011 Ottobrunn/Riemerling (DE); Wernicke, Hans Jürgen, Dr. Dipl.-Chem., D-8192 Geretsried (DE)
(74) Vertreter: Reitzner, Bruno, Dr.

(56) Entgegenhaltungen:
- EP-A- 0 063 034
- EP-A- 0 068 603
- DE-A- 1 542 451
- FR-A- 2 217 408
- GB-A- 2 079 737
- GB-A- 2 127 036
- US-A- 3 760 024
- US-A- 4 025 572

## Beschreibung

Die Erfindung betrifft Katalysatoren auf der Basis von kristallinen Alumosilikaten vom Pentasiltyp mit einem Si/Al-Atomverhältnis von mindestens 10.

Katalysatoren auf der Basis von kristallinen Alumosilikaten, die aus einer Aluminiumquelle, einer Siliciumquelle, einer Alkaliquelle, einem Templat (z.B. einer Tetrapropylammonium-Verbindung) und Wasser hergestellt werden, sind aus der US-PS 37 02 886 bekannt. Die Größe der Primärkristallite liegt je nach der Zusammensetzung der Ausgangsmischung bei 1 »m oder darunter. Es wurde nicht erkannt, daß die Größe der Primärkristallite einen bestimmten Grenzwert nicht unterschreiten darf. Zum Agglomerieren der Primärkristallite können Bindemittel verwendet werden, wobei jedoch über die Größe der Agglomerate keine Angaben gemacht sind und Aluminiumoxid nicht als Bindemittel genannt ist. Es finden sich auch keine Angaben über die Teilchengröße des Bindemittels. Wird Bentonit als Bindemittel verwendet, so haben die Katalysatoren eine ungünstige Porenverteilung und eine niedrigere Lebensdauer.

Aus der DE-A- 28 22 725 ist die Herstellung von Methanolumwandlungskatalysatoren auf der Basis von kristallinen Alumosilikaten bekannt. Der Durchmesser der Primärkristallite liegt bei 1 »m und mehr. Bezweckt wird die Herstellung von Primärkristalliten mit Durchmessern von weit über 1 »m. Dazu muß das Kristallwachstum durch höhere Temperaturen gefördert und die Keimbildung durch geringe Konzentrationen der für die Kristallisation der Zeolithe wesentlichen Template gehemmt werden. Außerdem finden sich keine Hinweise auf die Verwendung von Bindemitteln sowie über die Größe der Agglomerate.

Nach der DE-A- 24 05 909 (FR-A- 2 217 408) werden Katalysatoren zur Kohlenwasserstoffumwandlung auf der Basis von Zeolithen von ZSM-5-Typ hergestellt, wobei die mittleren Durchmesser der Primärkristallite im Bereich von 0,05 bis 0,1 »m liegen. Auf diese Weise soll die Alterung der Katalysatoren gegenüber Vergleichs Katalysatoren mit Primärkristallit-Durchmessern um 0,5»m verzögert werden. Die kleinen Primärkristallite, erzeugt durch Einstellen einer hohen Rührgeschwindigkeit, lassen sich schlecht filtrieren. Aus den Primärkristalliten werden Agglomerate in der Größenordnung von 0,1 bis 1 »m hergestellt. Zur Herstellung der Katalysatoren werden Die Agglomerate u.a. mit Aluminiumoxid als Bindemittel versetzt, wobei aber auch andere Bindemittel als gleichwertig angegeben sind. Über die Teilchengröße der Agglomerate in den fertigen Katalysatoren sowie der Bindemittel finden sich keine Angaben. Weiterhin wurde die Synthese in Anwesenheit von Schwefelsäure und unter Verwendung von Al₂ (SO₄)₃ · xH₂0 durchgeführt.

Nach der DE-A- 29 35 123 werden ZSM-5- oder ZSM-11-Zeolithe unter Verwendung von Ammoniumhydroxid und einem Alkohol als Templat hergestellt, wobei das Vorhandensein von Keimen kennzeichnend ist. Die Zeolithe werden als Crack- und Hydrocrack-Katalysatoren sowie als Katalysatoren für die Isomerisierung und das Entwachsen verwendet. Als Bindemittel kann Aluminiumoxid verwendet werden. Es finden sich jedoch keine Angaben über die Größe der Primärkristallite sowie der Agglomerate und des Bindemittels.

Ein Verfahren zur Herstellung von Zeolithen und ihre Verwendung als Katalysatoren für die Umwandlung von aliphatischen Verbindungen in aromatische Kohlenwasserstoffe ist in der DE-A-29 13 552 beschrieben. Als Templat wird ein Gemisch aus Butanol und Ammoniumhydroxid verwendet. Die Größe der Primärkristallite soll weniger als 3 »m, vorzugsweise weniger als 2 »m betragen. Eine Untergrenze ist nicht angegeben. Als Bindemittel für die Agglomerate kann u.a. Aluminiumoxid verwendet werden, wobei aber keine Teilchengrößen für die Agglomerate und das Bindemittel angegeben sind.

Aus der DE-A-35 37 459 ist ein Verfahren zur Herstellung großer, ebenmäßig ausgebildeter Kristalle von Zeolithen des Pentasil-Typs aus Si0₂ und einer Verbindung eines oder mehrerer dreiwertiger Elemente, wie Al, B, Fe, Ga, Cr in aminhaltigen Lösungen bekannt, das dadurch gekennzeichnet ist, daß man als Si0₂-haltigen Ausgangsstoff hochdisperses Si0₂, das durch Verbrennung einer Siliciumchlorid-Verbindung hergestellt wurde, verwendet. Die Zeolithe werden für die Umwandlung organischer Verbindungen, insbesondere für die Umwandlung von Methanol zu Kohlenwasserstoffen, die niedere Olefine und Aromaten enthalten, verwendet. Die erhaltenen Zeolithe sind nicht zu Agglomeraten verwachsen.

Die EP-A-173 901 betrifft ein Verfahren zur Herstellung von kleinen Zeolith-Kristalliten vom ZSM-5-Typ mit einem Si0₂/Al₂0₃-Molverhältnis von mehr als 5, entsprechend einem Si/Al-Atomverhältnis von mehr als 2,5. Die kleinste Abmessung der Kristallite liegt unterhalb 0,3 »m. Die Kristallite werden einer Ionenaustauschreaktion unterzogen und nach dem Vermischen mit einem Matrixmaterial zu größeren Teilchen geformt. Diese werden getrocknet und calciniert, wobei Katalysatoren für verschiedene Kohlenwasserstoff-Umwandlungsreaktionen erhalten werden. Über die Art und die Wirkung des Matrixmaterials finden sich keine Angaben.

Die EP-A-123 449 beschreibt ein Verfahren zur Umwandlung von Alkohol bzw. Ethern in Olefine unter Verwendung von mit Dampf behandelten Zeolith-Katalysatoren; diese haben eine Kristallgröße von weniger als 1 »m und können in eine Matrix eingebaut werden. Als Matrixmaterialien sind Tone, Kieselsäure und/oder Metalloxide angegeben.

Die US-A-42 06 085 betrifft Kohlenwasserstoff-Konvertierungskatalysatoren auf der Basis von Zeolithen und einem Matrixmaterial zur Erhöhung der Abriebfestigkeit. Als Matrixmaterial wird Aluminiumoxid aus Pseudoboehmit und Si0₂ aus Ammoniumpolysilikat oder Kieselsäuresol verwendet. Der bevorzugte Zeolith gehört dem Faujasit-Typ an. Über die Größe der Zeolithkristalle finden sich keine Angaben.

Der Erfindung liegt die Aufgabe zugrunde, Katalysatoren auf der Basis von kristallinen hochaktiven Alumosilikaten vom Pentasil-Typ mit einem Si/Al-Atomverhältnis von mindestens 10 zu schaffen, die eine verbesserte Lebensdauer sowie eine erhöhte Aktivität und Selektivität bei katalytischen Prozessen, insbesondere bei Methanolumwandlungsprozessen und bei Olefinoligomerisierungsprozessen haben.

Diese Katalysatoren sind dadurch gekennzeichnet, daß sie aus Primärkristalliten mit einem mittleren Durchmesser von mindestens 0,1 »m und höchstens 0,9 »m aufgebaut sind, die zu mindestens 20% zu Agglomeraten von 5 bis 500 »m vereinigt sind, wobei die Primärkristallite bzw. Agglomerate durch feinteiliges Aluminiumoxid, das durch Hydrolyse von aluminiumorganischen Verbindungen erhältlich ist, miteinander verbunden sind, daß ihre BET-Oberfläche 300 bis 600 m²/g und ihr Porenvolumen (nach der Quecksilberporosimetrie bestimmt) 0,3 bis 0,8 cm³/g beträgt, daß er in der H-Form vorliegt und die Menge des feinteiligen Aluminiumoxids. Bindemittels 10 bis 40 Gew.-%, bezogen auf das Gewicht des Endproduktes, beträgt.

Wenn die Primärkristallite teilweise zu Agglomeraten vereinigt sind, so sind sie nur locker aneinander gebunden, wie zum Beispiel in Filterkuchen. Aus diesem können die Primärkristallite verhältnismäßig leicht, z.B. durch Dispergieren des Filterkuchens in einem wäßrigen Medium und durch Rühren der Dispersion, wiedererhalten werden.

Alle vorstehend angegebenen Parameter sind wesentlich, damit Katalysatoren mit einer hohen Aktivität, Selektivität und Lebensdauer erhalten werden.

So ist es wichtig, daß die Primärkristallite einen mittleren Durchmesser von mindestens 0,1 »m und höchstens 0,9 »m haben. Vorzugsweise liegt der mittlere Durchmesser der Primärkristallite im Bereich von 0,1 bis 0,6 »m. Wenn der mittlere Durchmesser kleiner als 0,1 »m ist, vermindert sich die Lebensdauer der Katalysatoren beträchtlich, während bei einem Durchmesser von mehr als 0,9 »m die Anfangsaktivität sehr niedrig ist. Der mittlere Durchmesser der Primärkristallite wird definiert als das über eine Vielzahl von Kristalliten gemittelte arithmetische Mittel zwischen dem größten und dem kleinsten Durchmesser eines einzelnen Kristalliten. Diese Definition hat ihre Bedeutung bei Kristalliten mit einem unregelmäßigen Kristallhabitus, z.B. bei stäbchenförmigen Kristalliten. Bei kugelförmigen bzw. annähernd kugelförmigen Kristalliten fallen der größte und der kleinste Durchmesser zusammen.

Bei den angegebenen Werten für die Primärkristallite handelt es sich wieder um die mittleren Abmessungen (arithmetisches Mittel aus der größten und der kleinsten Abmessung, gemittelt über eine Vielzahl von Kristalliten).

Die Primärkristallite bzw. die Agglomerate sind durch feinteiliges Aluminiumoxid, das durch Hydrolyse von aluminiumorganischen Verbindungen erhältlich ist, miteiander verbunden.

Die Verbundkörper haben im allgemeinen Abmessungen von 20 bis 1000 »m, insbesondere von 50 bis 800 »m. Auch bei diesen Werten handelt es sich um mittlere Abmessungen, die wie vorstehend angegeben, definiert sind.

Der Aufbau des Katalysators aus Primärkristalliten, Agglomeraten und Bindemittelteilchen bestimmt auch die BET-Oberfläche (300 bis 600 m²/g), das Porenvolumen (0,3 bis 0,8 cm³/g) sowie den Porendurchmesser, d.h. mindestens 60 % der Poren haben vorzugsweise einen Durchmesser von 14 bis 90 nm.

Die BET-Oberfläche, das Porenvolumen und der Porendurchmesser stellen eine optimale Auswahl dar, um Katalysatoren mit hoher Aktivität, Selektivität und Lebensdauer zu erhalten.

Vorzugsweise liegt das feinteilige Aluminiumoxid-Bindemittel im Reaktionsansatz als peptisierbares Aluminiumoxidhydrat vor, wovon mindestens 95 % der Teilchen (auf den mittleren Durchmesser bezogen) ≦ 55 »m sind. Das feinteilige Aluminiumoxid-Bindemittel ist vorzugsweise durch Hydrolyse von Aluminiumtrialkylen oder Aluminiumalkoholaten erhältlich.

Der erfindungsgemäße Katalysator ist vorzugsweise dadurch gekennzeichnet, daß er auf folgende Weise erhältlich ist:
(a) in einem wäßrigen Reaktionsansatz, enthaltend eine Siliciumquelle, eine Aluminiumquelle, eine Alkaliquelle und ein Templat, wird bei erhöhter Temperatur und gegebenenfalls bei erhöhtem Druck in an sich bekannter Weise ein alkalisches Alumosilicatgel erzeugt und in ein kristallines Alumosilicat umgewandelt, wobei aber die Reaktion abgebrochen wird, wenn die erhaltenen Primärkristallite einen mittleren Durchmesser von mindestens 0,1 »m und höchstens 0,9 »m, vorzugsweise von 0,1 bis 0,6 »m haben;
(b) die Primärkristallite werden aus dem wäßrigen Reaktionsmedium als Voragglomerate abgetrennt, getrocknet und einer Zwischencalcinierung unterzogen;
(c) das Produkt von Stufe (b) wird zum Austausch der Alkaliionen in wäßrigem Medium mit einer protonenhaltigen oder beim Erhitzen Protonen liefernden Substanz umgesetzt, abgetrennt, getrocknet, und erneut einer Zwischencalcinierung unterzogen, worauf eine Agglomeratfraktion von etwa 5 bis 500 »m abgetrennt wird;
(d) die Agglomeratfraktion von Stufe (c) wird mit dem feinteiligen Aluminiumoxidhydrat vermischt;
(e) das Produkt von Stufe (d) wird einer Abschlußcalcinierung unterzogen.

Die Bedeutung der einzelnen Stufen, nach denen der erfindungsgemäße Katalysator erhältlich ist, ist nachstehend näher erläutert:
In der Stufe (a) wird zunächst ein wäßriger Reaktionsansatz, enthaltend eine Siliciumquelle (beispielsweise kolloidale Kieselsäure oder ein Alkalisilikat), eine Aluminiumquelle (beispielsweise Aluminiumhydroxid oder Natriumaluminat), eine Alkaliquelle (beispielsweise ein Alkalihydroxid, wobei die Alkaliquelle bei Verwendung von Alkalisilicaten auch ein Teil der Siliciumquelle und bei Verwendung von Alkalialuminaten auch ein Teil der Aluminiumquelle sein kann) und ein Templat, hergestellt. Die Gewichtsanteile zwischen Siliciumquelle und Aluminiumquelle werden so gewählt, daß kristalline Alumosilikate mit einem Si/Al-Atomverhältnis von mindestens 10, vorzugsweise von etwa 20 bis 500 : 1 erhalten werden. Aus dem Reaktionsansatz wird bei erhöhter Temperatur und gegebenenfalls bei erhöhtem Druck in an sich bekannter Weise ein alkalisches Alumosilikatgel erzeugt. Man kann schon bei Temperaturen ab 90°C arbeiten, doch werden in diesem Fall die Reaktionszeiten verhältnismäßig lang (etwa 1 Woche). Es wird deshalb vorzugsweise bei Temperaturen von 90 bis 190°C, insbesondere von 90 bis 150°C gearbeitet, wobei sich bei Temperaturen von mehr als 100°C (unter Normalbedingungen) in Abhängigkeit von der Temperatur automatisch ein Überdruck einstellt.

Das Alumosilikatgel wandelt sich im Laufe der Reaktion in ein kristallines Alumosilikat um. Wenn die Temperatur des Reaktionsansatzes höher als 190°C liegt, wird das Wachstum der Alumosilikat-Primärkristallite zu schnell, und es werden Primärkristallite mit einem Durchmesser von mehr als 0,9 »m erhalten, während gleichzeitig noch Alumosilicatgel im Reaktionsansatz vorhanden ist.

Als Template werden Tetraalkylammoniumverbindungen, vorzugsweise Tetrapropylammoniumhydroxid (TPAOH) oder Tetrapropylammoniumbromid (TPABr) eingesetzt. Man kann als Template auch Gemische aus Ammoniak oder einem organischen Amin und einer weiteren organischen Verbindung aus der Gruppe der Alkohole, vorzugsweise Butanol, verwenden.

Der wäßrige Reaktionsansatz von Stufe (a) hat vorzugsweise einen pH-Wert von 10 bis 13. Bei einem pH-Wert von weniger als 10 verläuft die Umwandlung des Alumosilikatgels in das kristalline Alumosilikat verhältnismäßig langsam. Bei höheren pH-Werten als 13 können sich die Alumosilikatkristalle in einigen Fällen wieder auflösen. Dies kann aber im allgemeinen toleriert werden, weil sich in der Regel zunächst nur die kleineren Primärkristallite mit einem Durchmesser von weniger als 0,1 »m wieder auflösen.

Die Bildung der kristallinen Alumosilikat-Primärkristallite kann durch geeignete Auswahl der Siliciumquelle, der Aluminiumquelle, der Alkaliquelle und des Templats sowie durch geeignete Auswahl der Temperatur und des pH-Werts und der Rührgeschwindigkeit geregelt werden. Wesentlich ist, daß die Reaktion abgebrochen wird, wenn die erhaltenen Primärkristallite einen mittleren Durchmesser von mindestens 0,1 »m und höchstens 0,9 »m haben.

Zu diesem Zweck werden mehrere Testansätze durchgeführt. Schon nach wenigen Versuchen lassen sich die optimalen Parameter ermitteln, aufgrund derer die erforderlichen Größenbereiche der Primärkristallite erreicht werden. Ein Indiz für die Beendigung der Reaktion besteht auch darin, daß der pH-Wert des Reaktionsansatzes sprunghaft ansteigt.

Erfindungsgemäß braucht nicht in jedem Fall ein neuer Reaktionsansatz hergestellt zu werden. Vielmehr kann zur Erzeugung des Alumosilicatgels die Siliciumquelle, die Alkaliquelle, die Aluminiumquelle, das Templat und das Wasser aus den Mutterlaugen vorheriger Synthesen verwendet und durch die für die Synthese des Alumosilicatgels erforderlichen Mengen der genannten Verbindungen ergänzt werden.

Die Bildung der Alumosilicat-Primärkristallite von Stufe (a) erfolgt vorzugsweise bei einem pH-Wert zwischen 10 und 13, wobei der Reaktionsansatz gerührt wird. Auf diese Weise wird die Größenverteilung der Primärkristallite homogenisiert. Die Rührgeschwindigkeit soll aber vorzugsweise nicht mehr als 900 U/min betragen. Bei größeren Rührgeschwindigkeiten ist der Anteil der kleineren Primärkristallite höher, so daß die Reaktionszeit verlängert werden muß, damit gewährleistet ist, daß der mittlere Durchmesser aller Primärkristallite mindestens 0,1 »m beträgt.

In der Stufe (b) werden die Primärkristallite aus dem wäßrigen Reaktionsmedium als Voragglomerate abgetrennt, d.h. nicht als Einzelkristallite. Dies wird vorzugsweise dadurch erreicht, daß man dem wäßrigen Reaktionsmedium ein Flockungsmittel zusetzt. Im allgemeinen wird als Flockungsmittel eine kationische organische makromolekulare Verbindung, vorzugsweise ein Copolymer aus Acrylamid und einem kationischen Acrylsäurederivat, verwendet.

Das Flockungsmittel erleichtert nicht nur die Abtrennung der Primärkristallite aus dem Reaktionsmedium (verbesserte Filtrierbarkeit), sondern bewirkt auch, daß sich die Primärkristallite zu Voragglomeraten zusammenschließen, die hinsichtlich Größe, Struktur und Anlagerung der Primärkristallite schon weitgehend den in der folgenden Stufe gebildeten Agglomeraten gleichen. Die Voragglomerate werden getrocknet und einer Zwischencalcinierung unterzogen, die zunächst vorzugsweise in einer inerten Atmosphäre bei etwa 200 bis 350°C, insbesondere bei etwa 250°C durchgeführt wird, wobei ein Teil des Templats desorbiert wird.

Die Zwischencalcinierung kann dann in einer oxidierenden Atmosphäre bei etwa 500 bis 600°C vervollständigt werden, wobei die gegebenenfalls noch vorhandene Restmenge an Templat abgebrannt wird.

Im allgemeinen werden die Voragglomerate etwa 1 bis 20 Stunden in der inerten Atmosphäre und etwa 1 bis 30 Stunden in der oxidierenden Atmosphäre zwischencalciniert.

In der Stufe (c) wird das Produkt von Stufe (b) zum Austausch der Alkaliionen in wäßrigem Medium mit einer protonenhaltigen oder beim Erhitzen Protonen liefernden Substanz umgesetzt. Beispielsweise kann der Ionenaustausch mit Hilfe einer verdünnten Mineralsäure (z.B. Salzsäure oder Schwefelsäure) oder einer organischen Säure (z.B. Essigsäure) durchgeführt werden. Der Ionenaustausch erfolgt vorzugsweise unter Rühren mindestens eine Stunde bei Temperaturen zwischen 25 und 100°C, wobei zumindest ein Teil der Alkaliionen in den Voragglomeraten der Primärkristallite durch Wasserstoffionen ausgetauscht werden. Falls erforderlich, kann der Ionenaustausch unter den gleichen Bedingungen wiederholt werden.

Nach dem Austausch der Alkaliionen im wäßrigen Medium wird das Protonen enthaltende Produkt (H-Zeolith) abgetrennt (beispielsweise durch Filtration), getrocknet und erneut einer Zwischencalcinierung unterzogen. Die Zwischencalcinierung wird bei Temperaturen von 400 bis 800°C, vorzugsweise bei etwa 600°C über einen Zeitraum von 5 bis 20 Stunden durchgeführt.

Statt mit der verdünnten Säure kann der Ionenaustausch auch mit Hilfe einer Ammoniumsalzlösung unter vergleichbaren Bedingungen durchgeführt werden. In diesem Fall werden die Alkaliionen durch Ammoniumionen ausgetauscht. Wird das so erhaltene Produkt zwischencalciniert, so wird Ammoniak entfernt, und man erhält ein Protonen enthaltendes Produkt.

Das nach der Zwischencalcinierung erhaltene Produkt enthält einerseits Agglomerate, die ≧ 500 »m sind, und andererseits Staubanteile, die ≦ 5 »m sind. Es wird daher eine Agglomeratfraktion von etwa 5 bis 500 »m abgetrennt.

Diese Agglomeratfraktion wird in der Stufe (d) mit dem feinteiligen Aluminiumoxidhydrat vermischt, von dem mindestens 95 % ≦ 55 »m und mindestens 30 % ≧ 35 »m sind. Diese Werte sind, gemittelt über eine Vielzahl von Kristalliten, jeweils auf den mittleren Durchmesser bezogen, der wie der mittlere Durchmesser der Primärkristallite definiert ist. Im einzelnen hat das Aluminiumoxid typischerweise folgendes Kornspektrum.
99 % ≦ 90 »m
95 % ≦ 45 »m
55 % ≦ 25 »m.

Das Aluminiumoxidhydrat ist im wesentlichen für die Einstellung des Porenvolumens des erfindungsgemäßen Katalysators verantwortlich. Die Menge des feinteiligen Aluminiumoxidhydrat-Bindemittel beträgt 10 bis 40 Gew.-%, bezogen auf das Gesamtgewicht des Produktes von Stufe (d). Vorzugsweise ist das feinteilige Aluminiumoxidhydrat-Bindemittel peptisierbares Aluminiumoxid, das besonders Na- und Fe-arm ist.

Die Peptisierung erfolgt vorzugsweise mit einer stark verdünnten, schwachen Säure, z.B. in 1,5-%iger Essigsäure.

Das Produkt von Stufe (d) wird einer Abschlußcalcinierung unterzogen. Diese kann bei Temperaturen von etwa 400 bis 800°C, vorzugsweise bei etwa 540°C, über einen Zeitraum von 5 bis 20 Stunden durchgeführt werden.

Das so erhaltenen Endprodukt kann in Methanolumwandlungsprozessen zur Herstellung von Olefinen und/oder Benzin bzw. zur Herstellung von Benzin und/oder Dieselkraftstoffen aus niedermolekularen Olefinen oder niedermolekularen organischen Sauerstoffverbindungen, einschließlich niederen Alkoholen, wie Methanol oder Äthanol und Dimethyläther, verwendet werden.

Die Erfindung ist durch die nachstehenden Beispiele erläutert.

### Vergleichsbeispiele 1 und 2

Eine Reaktionsmischung wurde durch inniges Mischen zweier Lösungen bei Raumtemperatur in einem 40-Liter-Autoklaven hergestellt. Die beiden Lösungen wurden als Lösung A und Lösung B bezeichnet. Die Lösung A wurde hergestellt, indem in 11 kg deionisiertem Wasser 554 g Tetrapropylammoniumbromid (TPABr) gelöst wurden. In diese Lösung wurden 2500 g einer handelsüblichen Kieselsäure eingetragen. Die Lösung B wurde hergestellt, indem in 5,5 Liter deionisiertem Wasser 183 g NaOH (Beispiel 2: 116,5 NaOH) und anschließend 34,1 g NaAl0₂ gelöst wurden. Die noch warme Lösung B wurde zur Lösung A gegeben. Der Autoklav wurde dann geschlossen und unter Rühren mit etwa 60 U/min sofort auf die Reaktionstemperatur gebracht. Nach etwa 137 Stunden (Beispiel 2: 240 Stunden) war die Reaktion abgeschlossen, wie aus dem pH-Sprung zu ersehen war (von 11,4 auf 12,0). Nach dem Abkühlen wurde der Autoklav geöffnet, das Produkt dem Reaktionskessel entnommen und filtriert. Der gut filtrierbare Filterkuchen wurde bis zu einem pH-Wert von 7 bis 8 und bis zu einer Br⁻ -Konzentration im Waschwasser von weniger als 1 ppm gewaschen und bei 540°C 24 Stunden calciniert. Die Größe der Primärkristallite ist in Tabelle I angegeben. In Reihenversuchen waren vorher die zur Erzielung der angestrebten Kristallitgrößen erforderlichen Bedingungen ermittelt worden.

Die Umsetzung wurde in den vorliegenden Vergleichsbeispielen bewußt sehr lange durchgeführt, um größere Primärkristallite zu erhalten.

Der calcinierte Na-Zeolith wurde in der 5-fachen Menge einer 1-molaren wäßrigen HCl-Lösung suspendiert und auf 80°C gebracht. Bei dieser Temperatur wurde eine Stunde gerührt.

Der H-Zeolith wurde bis zu einem Chloridgehalt im Waschwasser von weniger als 5 ppm gewaschen. Der getrocknete H-Zeolith wurde mit einem kommerziellen Granulator auf 2 mm zerkleinert und an der Luft mit einer Aufheizrate von 1°C/min. auf 540°C gebracht und bei dieser Temperatur an der Luft 10 Stunden calciniert. Die physikalischen und chemischen Eigenschaften des calcinierten Zeolithen in der Protonen-Form (H-Zeolith) ist in Tabelle I angegeben.

5000 g des calcinierten H-Zeolithen wurden mit einer Labormühle gemahlen. Das Korngrößenspektrum des gemahlenen Zeoliths war wie folgt:
0,2 Gew.-% > 500 »m
20 Gew.-% = 200 bis 500 »m
39 Gew.-% = 100 bis 200 »m
23 Gew.-% = 40 bis 100 »m
17 Gew.-% < 40 »m.

Der gemahlene Zeolith wurde in einem Kneter mit 1250 g eines Calciumbentonits mit folgender Korngrößenverteilung
98 Gew.-% ≦ 150 »m
88 Gew.-% ≦ 75 »m
80 Gew.-% ≦ 60 »m
15 Min. trocken gemischt. Zu dieser Mischung wurden langsam etwa 190 ml Steatitöl (zur Erleichterung des Verknetens) und 3770 ml Wasser gegeben. Diese Mischung wurde etwa 30 Min. bis zur Plastifizierung geknetet. Die Mischung wurde mit Hilfe eines Extruders zu Formkörpern mit einem Durchmesser von etwa 1,5 mm bis etwa 3,2 mm und einer Länge von etwa 2 mm bis etwa 6 mm extrudiert. Die Analysenergebnisse sind in Tabelle I angegeben.

### Vergleichsbeispiel 3

Nach diesem Beispiel wurden Alumosilikat-Zeolithe mit einer Primärkristallitgröße von < 1 »m hergestellt. Die Katalysatoren wurden unter Zusatz von Bentonit als Bindemittel hergestellt. Im einzelnen wurde wie folgt verfahren:
Eine Reaktionsmischung wurde durch inniges Mischen zweier Lösungen bei Raumtemperatur in einem 40 Liter-Autoklaven hergestellt. Die beiden Lösungen wurden als Lösung A und Lösung B bezeichnet. Die Lösung A wurde hergestellt, indem in 11 kg deionisiertem Wasser 2218 g TPABr gelöst wurden. In diese Lösung wurden 5000 g einer handelsüblichen Kieselsäure eingetragen. Die Lösung B wurde hergestellt, indem in 5,5 Liter deionisiertem Wasser 766 g NaOH und anschließend 45,6 g NaAlO₂ gelöst wurden. Die noch warme Lösung B wurde zur Lösung A gegeben. Der Autoklav wurde dann geschlossen und unter Rühren bei etwa 60 U/min. sofort auf die Reaktionstemperatur gebracht. Nach etwa 50 Stunden war die Reaktion beendet, wie aus dem pH-Sprung zu ersehen war. Nach dem Abkühlen wurde der Autoklav geöffnet, das Produkt dem Reaktionskessel entnommen und filtriert. Der Filterkuchen wurde in etwa 40 Liter deionisiertem Wasser aufgeschlämmt, mit etwa 5 Liter einer 0,4 Gew.-%igen wäßrigen Suspension eines handelsüblichen kationischen Flockungsmittels (Praestol BC 11L, Copolymer aus Acrylamid und einem kationischen Acrylsäurederivat) versetzt und nach dem Rühren und Absetzen der Voragglomerate des Feststoffes dekantiert. Der beschriebene Waschprozeß wurde wiederholt, bis das Waschwasser einen pH-Wert von 7 bis 8 und eine Br-Konzentration von weniger als 1 ppm hatte. Die Aufschlämmung, in der Voragglomerate von Primärkristalliten zu erkennen waren, die offenbar durch das Flockungsmittel zusammengehalten wurden, wurde in der in den Vergleichsbeispielen 1 und 2 angegebenen Weise filtriert. Der Filterkuchen wurde anschließend bei 120°C 12 Stunden getrocknet.

Der getrocknete Filterkuchen wurde mit einem handelsüblichen Granulator auf eine Korngröße von 2 mm zerkleinert.

Das Granulat wurde mit einer Aufheizrate von 1°C/min. unter Stickstoff (1000 Nl/h) auf 350°C gebracht und bei 350°C 15 Stunden unter Stickstoff (1000 Nl/h) calciniert. Dann wurde die Temperatur mit einer Aufheizrate von 1°C/min auf 540°C erhöht, und das Granulat wurde 24 Stunden bei dieser Temperatur an Luft calciniert, um das restliche TPABr abzubrennen. Der calcinierte Na-Zeolith wurde analysiert, wobei die in Tabelle I angegebenen Ergebnisse erhalten wurden.

Der calcinierte Na-Zeolith wurde in der 5-fachen Menge einer 1-molaren wäßrigen HCl-Lösung suspendiert und auf 80°C gebracht. Bei dieser Temperatur wurde eine Stunde gerührt. Dann wurde etwa 1 Liter einer 0,4 Gew.-%igen Suspension des kationischen Flockungsmittels von Beispiel 1 hinzugegeben, und die überstehende Säure wurde nach Absitzen des Feststoffes abdekantiert. Der so beschriebene Vorgang wurde noch einmal wiederholt.

Der Feststoff wurde in etwa 10 Waschvorgängen jeweils in 60 Liter deionisiertem Wasser unter Rühren suspendiert und mit durchschnittlich 100 ml einer 0,4 Gew.-%igen Suspension des Flockungsmittels versetzt. Nach dem Absitzen des Zeolithen wurde die überstehende Lösung dekantiert. Als der Gehalt an Cl⁻ im Waschwasser < 5 ppm war, wurde die Suspension abfiltriert und bei 120°C 15 Stunden getrocknet.

Der getrocknete H-Zeolith wurde mit einem handelsüblichen Granulator auf 2 mm zerkleinert und unter Luft mit einer Aufheizrate von 1°C/min auf 540°C gebracht und bei dieser Temperatur unter Luft 10 Stunden calciniert. Die Spezifikation dieses calcinierten H-Zeolithen ist in Tabelle I angegeben.

5000 g des calcinierten H-Zeolithen wurden mit einer Labormühle auf eine Korngröße von 500 »m gemahlen und in einem Kneter mit 1250 g eines Calciumbentonits mit einem Teilchengrößenspektrum von
98 Gew.-% ≦ 150 »m
88 Gew.-% ≦ 75 »m
80 Gew.-% ≦ 60 »m
15 Min. trocken gemischt. Zu dieser Mischung wurden langsam 190 ml Steatitöl und 3770 ml Wasser gegeben. Diese Mischung wurde etwa 30 min. bis zur Plastifizierung geknetet. Das Muster wurde mit Hilfe eines Extruders zu Formkörpern mit einem Durchmesser von etwa 3,2 mm und einer Länge von etwa 6 mm extrudiert.

Die Ergebnisse der Analyse des Produkts sind in Tabelle I angegeben.

### Beispiel 1

Die Alumosilikatkristalle werden wie im Vergleichsbeispiel 3 beschrieben, hergestellt. Auch die Calcinierung und der Ionenaustausch erfolgte gemäß Vergleichsbeispiel 3.

5000 g eines calcinierten H-Zeolithen wurden mit Hilfe einer Labormühle auf eine Korngröße von etwa 500 »m gemahlen und in einem Doppel-Z-Kneter mit 1470 g eines handelsüblichen peptisierbaren Aluminiumoxidhydrats mit einem Korngrößenspektrum von
99 Gew.-% ≦ 90 »m
95 Gew.-% ≦ 45 »m
55 Gew.-% ≦ 25 »m
15 Min. trocken gemischt. Zu dieser Mischung wurden langsam 4565 ml einer 1,5 Gew.-%igen wäßrigen Essigsäurelösung (zur Peptisierung des Aluminiumoxidhydrats) und 417 ml Steatitöl gegeben.

Diese Mischung wurde etwa 30 min. bis zur Plastifizierung geknetet und in einem handelsüblichen Extruder zu Formkörpern mit einem Durchmesser von etwa 1,6 mm und einer Länge von extrudiert. Die Abschlußcalcinierung wurde 3 Stunden bei 650°C durchgeführt.

Die Analysenwerte sowie die physikalischen und chemischen Eigenschaften des Produkts sind in Tabelle II angegeben.

### Beispiel 2

Eine Reaktionsmischung wurde durch inniges Mischen zweier Lösungen bei Raumtemperatur in einem 40-Liter Autoklaven hergestellt. Die beiden Lösungen wurden als Lösung A und Lösung B bezeichnet. Die Lösung A wurde hergestellt, indem in 11 kg deionisiertem Wasser 2218 g TPABr gelöst wurden. In diese Lösung wurden 5000 g einer handelsüblichen Kieselsäure eingetragen. Die Lösung B wurde hergestellt, indem in 5,5 Liter deionisiertem Wasser 766 g NaOH und anschließend 136,6 NaAlO₂ unter Rühren gelöst wurden. Die noch warme Lösung B wurde zur Lösung A gegeben. Der Autoklav wurde dann geschlossen und unter Rühren mit etwa 60 U/min sofort auf die Reaktionstemperatur von 130°C gebracht. Nach etwa 60 Stunden bei 130°C unter Rühren mit 60 U/min war die Reaktion beendet, wie aus dem pH-Sprung von 11,4 auf 12,3 und der anschließenden Konstanz des pH-Wertes zu ersehen war. Nach dem Abkühlen wurde der Autoklav geöffnet, das Produkt dem Reaktionskessel entnommen und filtriert. Gleichzeitig wurde die Größe der Primärkristallite bestimmt (vgl. Tabelle II). Der Filterkuchen wurde in etwa 40 Liter deionisiertem Wasser aufgeschlämmt, mit etwa 5 Liter einer 0,4 Gew.-%igen wäßrigen Suspension eines Flockungsmittels (Praestol BC 11L, Copolymer aus Acrylamid und einem kationischen Acrylsäurederivat) versetzt und nach dem Rühren und Absetzen des Feststoffes dekantiert. Der beschriebene Waschprozeß wurde bis zu einem pH-Wert von 7 bis 8 und bis zu einer Br-Konzentration im Waschwasser von weniger als 1 ppm fortgesetzt. Dann wurde der Feststoff wie oben beschrieben, filtriert. Der Filterkuchen wurde anschließend bei 120°C 12 Stunden getrocknet.

Die Herstellung des Katalysators erfolgte wie nach Beipiel 1. Die Größe der Primärkristallite sowie die chemischen und physikalischen Eigenschaften des Katalysators sind in Tabelle II angegeben.

### Beispiel 3

Eine Reaktionsmischung wurde durch inniges Mischen zweier Lösungen bei Raumtemperatur in einem 40-Liter-Autoklaven hergestellt. Die beiden Lösungen wurden als Lösung A und Lösung B bezeichnet. Die Lösung A wurde hergestellt, indem etwa 1,92 kg deionisiertes Wasser mit 2,537 Liter einer 20 Gew.-%igen wäßrigen Tetrapropylammoniumhydroxid (TPAOH)-Lösung gemischt wurden. Dieser Lösung wurden 1500 g einer handelsüblichen Kieselsäure eingetragen. Die Lösung B wurde hergestellt, indem in 1 Liter deionisiertem Wasser 120 g NaOH und anschließend 14,3 g NaAlO₂ gelöst wurden. Die noch warme Lösung B wurde zu Lösung A gegeben. Der Autoklav wurde dann geschlossen und unter Rühren mit etwa 60 U/min sofort auf 130°C gebracht. Nach etwa 24 Stunden bei 130°C war die Reaktion beendet, wie aus dem pH-Sprung zu ersehen war. Nach dem Abkühlen wurde der Autoklav geöffnet und das Produkt dem Reaktionskessel entnommen und filtriert. Der Filterkuchen wurde, wie in Beispiel 2 beschrieben, gewaschen, calciniert und zum fertigen Katalysator aufgearbeitet. Die Größe der Primärkristallite sowie die chemischen und physikalischen Eigenschaften des Katalysators sind in Tabelle II angegeben.

### Beispiel 4

Eine Reaktionsmischung wurde durch inniges Mischen zweier Lösungen bei Raumtemperatur in einem 40-Liter-Autoklaven hergestellt. Die beiden Lösungen wurde als Lösung A und Lösung B bezeichnet. Die Lösung A wurde hergestellt, indem 988 g Butanol und 266 g 25%ige NH₃-Lösung mit 10 kg H₂0 vermischt wurden. Die Lösung B wurde hergestellt, indem in etwa 2 kg deionisiertem Wasser 160 g NaOH gelöst wurden. In dieser Lösung wurden 36,3 g NaAlO₂ eingetragen. Die Lösung A wurde zu Lösung B gegeben, und unter Rühren wurden in diese Mischung 2000 g einer handelsüblichen Kieselsäure eingetragen. Der Autoklav wurde dann geschlossen und unter Rühren mit etwa 64 U/min. sofort auf die Reaktionstemperatur von 180°C gebracht. Nach etwa 34 Stunden war die Reaktion abgeschlossen, wie aus dem pH-Sprung zu ersehen war. Nach dem Abkühlen wurde der Autoklav geöffnet, und das Produkt wurde dem Reaktionskessel entnommen und filtriert. Der Filterkuchen wurde nach dem im Beispiel 2 beschriebenen Verfahren zum fertigen Katalysator aufgearbeitet. Die Größe der Primärkristallite sowie die physikalischen und chemischen Eigenschaften des Katalysators sind in Tabelle II angegeben.

**Tabelle I**

| Vergleichsbeispiel | 1 | 2 | 3 |
|---|---|---|---|
| Molverhältnis der Ausgangsstoffe | | | |
| Si0₂ | 100 | 100 | 100 |
| NaAl0₂ | 1 | 1 | 0,67 |
| NaOH | 11 | 7 | 23 |
| TPABr | 5 | 5 | 10 |
| H₂0 | 2200 | 2200 | 1100 |

| Kristallisationsdaten | | | |
|---|---|---|---|
| Temp.(°C) | 130 | 130 | 130 |
| Zeit (h) | 137 | 240 | 50 |

| Si- und Al-Gehalt der Elementarzelle des Na-Zeolithen | | | |
|---|---|---|---|
| Si | 95,14 | 95,22 | 94,98 |
| Al | 0,86 | 0,77 | 1,02 |
| Si/Al | 111,26 | 123 | 93 |

| Phys. und chem.Eigenschaften des calcinierten Zeolithen in der Protonenform | | | |
|---|---|---|---|
| Si/Al | 130 | 130 | 84 |
| Kristallinität (%) | 100 | 100 | 100 |
| Primärkristallitgröße (»m) | 2,75 | 4,5 | 0,4 |
| BET-Oberfläche (m²/g) | 318 | 345 | 320 |
| Porenvolumen (cm³/g) | 0,38 | 0,45 | 0,52 |
| Poren größer 80 nm (%) | 91,4 | 72,5 | 78,8 |
| Poren 14 - 80 nm (%) | 4,3 | 14,6 | 15,9 |

**Tabelle II**

| Beispiel | 1 | 2 | 3 | 4 |
|---|---|---|---|---|
| Molverhältnis der Ausgangsstoffe | | | | |
| Si0₂ | 100 | 100 | 100 | 100 |
| NaAl0₂ | 0,67 | 2 | 0,67 | 1,33 |
| NaOH | 23 | 23 | 12 | 12 |
| TPABr | 10 | 10 | | |
| TPAOH | | | 10 | |
| (0,4BuOH, 0,1 NH₃) | | | | 100 |
| H₂0 | 1100 | 1100 | 1100 | 2000 |

| Kristallisationsdaten | | | | |
|---|---|---|---|---|
| Temp.(°C) | 130 | 130 | 130 | 180 |
| Zeit (h) | 50 | 60 | 24 | 34 |
| Kristallinität (%) | 100 | 100 | 100 | 100 |
| Primärkristallitgröße (»m) | 0,3 | 0,3 | 0,3 | 0,5 |

| Si- und Al-Gehalt der Elementarzelle des Na-Zeolithen | | | | |
|---|---|---|---|---|
| Si | 95,04 | 93,6 | 95,04 | 94,61 |
| Al | 0,953 | 1,03 | 0,96 | 1,39 |
| Si/Al | 99,7 | 39,07 | 99 | 66 |

| Phys. und chem.Eigenschaften des Katalysators | | | | |
|---|---|---|---|---|
| Si/Al | 105 | 41 | 104 | 70 |
| BET-Oberfläche (m²/g) | 366 | 370 | 375 | 365 |
| Porenvolumen (cm³/g) | 0,51 | 0,6 | 0,55 | 0,45 |
| Poren ≧ 80 nm (%) | 21,1 | 8,6 | 10,0 | 15,0 |
| Poren 14 - 80 nm (%) | 68,0 | 76,5 | 61,0 | 63,0 |

### Beispiel 5

Eine Reaktionsmischung wurde durch inniges Mischen zweiter Lösungen bei Raumtemperatur in einem 40-Liter-Autoklaven hergestellt. Die beiden Lösungen wurden als Lösung A und Lösung B bezeichnet. Die Lösung A wurde hergestellt, indem in 11 kg deionisiertem Wasser 2218 g TPABr gelöst wurden. In dieser Lösung wurden 5000 g einer handelsüblichen Kieselsäure (Si0₂ x 0,45 H₂0) eingetragen. Die Lösung B wurde hergestellt, indem in 5,5 Liter deionisiertem Wasser 766 g NaOH und anschließend 45,6 g NaAlO₂ unter Rühren gelöst wurden. Die noch warme Lösung B wurde zur Lösung A gegeben. Der Autoklav wurde dann geschlossen und unter Rühren mit etwa 60 U/min sofort auf die Reaktionstemperatur von 130°C gebracht. Nach etwa 70 Stunden bei 130°C war die Reaktion beendet, wie aus dem pH-Sprung zu ersehen war. Nach dem Abkühlen wurde der Autoklav geöffnet, und das Produkt wurde dem Reaktionskessel entnommen und filtriert. Die physikalischen und chemischen Eigenschaften sind in Tabelle III angegeben.

Die Mutterlauge (9 Liter) enthielt 0,69 mol/Liter OH⁻, 2 mg/Liter Al, 62,7 g/Liter C (aus dem Templat), 35 g/Liter Si und 51,1 g/Liter Br⁻ (aus dem Templat).

Der zweite Ansatz mit der gleichen molaren Zusammensetzung wurde durch inniges Mischen zweiter Lösungen bei Raumtemperatur in einem 40-Liter-Autoklaven hergestellt. Die beiden Lösungen wurden als Lösung C und Lösung D bezeichnet. Die Lösung C wurde hergestellt, indem in 9 kg deionisiertem Wasser 520 g NaOH und 45,6 g NaAlO₂ gelöst wurden. Zu dieser Lösung wurden 1043 g TPABr hinzugefügt. Für die Lösung D wurde die Mutterlauge aus dem ersten Ansatz verwendet. Die Lösung D wurde zur Lösung C gegeben. In diese Mischung wurden 5000 g handelsübliche Kieselsäure eingetragen. Der Autoklav wurde dann geschlossen und unter Rühren mit etwa 60 U/min sofort auf 130°C gebracht. Nach etwa 72 Stunden war die Reaktion beendet, wie aus dem pH-Sprung zu ersehen war. Nach dem Abkühlen wurde der Autoklav geöffnet, das Produkt dem Reaktionskessel entnommen und filtriert.

Für die weiteren Zyklen wurden unter Berücksichtigung der jeweiligen Zusammensetzung der Mutterlauge entsprechend verfahren. Der Filterkuchen wurde bis zu einem pH-Wert von 7 bis 8 und bis zu einer Br⁻-Konzentration im Waschwasser von weniger als 1 ppm unter Zusatz von Flockungsmittel, wie im Vergleichsbeispiel 3 beschrieben, gewaschen und bei 540°C 24 Stunden calciniert.

Der Katalysator wurde nach dem in Beispiel 1 beschriebenen Verfahren fertiggestellt. Die Ergebnisse sind in Tabelle III angegeben.

**Tabelle III**

| Beispiel 5 Molverhältnis der Ausgangsstoffe (Basis Si0₂ | Synthese | 1.Recycle | 5.Recycle |
|---|---|---|---|
| Si0₂ | 100 | 100 | 100 |
| NaAl0₂ | 0,76 | 0,67 | 0,67 |
| NaOH | 26,1 | 23 | 23,1 |
| TPABr | 11,3 | 10 | 10 |
| H₂0 | 1300 | 1250 | 1250 |

| Kristallisationsdaten | | | |
|---|---|---|---|
| Temp.(°C) | 130 | 130 | 130 |
| Zeit (h ) | 72 | 72 | 72 |
| Rühren (U/min) | 63 | 60 | 60 |
| Kristallinität (%) | 100 | 100 | 100 |
| Primärkristallitgröße (»m) | 0,2-0,8 | 0,2-0,8 | 0,2-0,8 |

| Si- und Al-Gehalt der Elementarzelle des Na-Zeolithen | | | |
|---|---|---|---|
| Si | 95,17 | 95,24 | 95,22 |
| Al | 0,83 | 0,76 | 0,78 |
| Si/Al | 114 | 125 | 122 |

| Phys. und chem.Eigenschaften des Katalysators | | | |
|---|---|---|---|
| Si/Al | 100 | 100 | 100 |
| BET-Oberfläche (m²/g) | 360 | 355 | 363 |
| Porenvolumen (cm³/g) | 0,49 | 0,53 | 0,48 |
| Poren ≧ 80 nm (%) | 14 | 7 | 13 |
| Poren 14 - 80 mm (%) | 62 | 67 | 65 |

### Anwendungsbeispiel 1

Dieses Anwendungsbeispiel zeigt anhand katalytischer Daten des CMO-Verfahrens (Conversion of Methanol to Olefins) in einem isothermen Festbettreaktor die erfindungsgemäß durch die Verwendung des Aluminiumoxidhydrat-Bindemittels und die dadurch bedingte Porenradienverteilung erreichbare Verbesserung.

Die Versuche wurden im einzelnen wie folgt durchgeführt:
Das Feed Methanol/Wasser (1 g/1 g) wurde mit einer LHSV von 1 (l/(lxh), d.h. Liter Gesamtfeed pro Liter Katalysator und pro Stunde bei einem Druck von 1 bar nach Passieren eines isothermen Festbettrohrreaktors zur teilweisen Konversion von Methanol in Dimethylether über 300 cm³ CMO-Katalysator in einem isothermen Festbettrohrreaktor geleitet. Die Umwandlung des Methanols wurde bei nahezu 100 % gehalten. Bei einem bestimmten Wert (Konversion EOR, %) wurde die Reaktion abgebrochen und der Katalysator regeneriert.

Gasphase und flüssige Phase am Ausgang des CMO-Katalysator-Reaktors wurden mit den üblichen gaschromatographischen Analysenverfahren bestimmt. Die Verteilung der Kohlenwasserstoffe ist in Tabelle IV zusammen mit anderen relevanten Daten zusammengefaßt.

**Tabelle IV**

| Katalysator Bindemittel Primärkrist.(»m) | Vergleichsbeispiel 1 Bentonit 2-3,5 | Vergleichsbeispiel 3 Bentonit 0,3 | Beispiel 1 Aluminiumoxid 0,3 |
|---|---|---|---|
| Temperatur (°C) | 415 | 415 | 400 |
| Druck (bar) | 1 | 1 | 1 |
| LHSV(l/(lxh)) | 1 | 1 | 1 |
| MeOH/H₂O(g/g) | 1 | 1 | 1 |
| Dauer 1. Zyklus(h) | 197 | 554 | 927 |
| Dauer 2. Zyklus(h) | 62 | 404 | 2000 |
| 1. Zyklus, Mittelwerte, | Gew.-% | | |
| C1-C4 Paraffine | 10,6 | 9,44 | 10,5 |
| C2-C4 Olefine | 56,5 | 56,6 | 51,9 |
| C5+Gasolin | 32,8 | 33,9 | 37,6 |
| Konversion EOR, % | 96,2 | 97,1 | 99,7 |
| 2. Zyklus, Mittelwerte, | Gew.-% | | |
| C1-C4 Paraffine | 10,5 | 6,68 | 7,48 |
| C2-C4 Olefine | 56,2 | 58,7 | 54,76 |
| C5+Gasolin | 33,2 | 34,6 | 37,76 |
| Konversion EOR, % | 99,97 | 97,1 | 100 abgebr. |

Tabelle IV zeigt eindeutig die erhöhte Lebensdauer des erfindungsgemäßen Katalysators von Beispiel 1. Der erste Zyklus wurde bei den Katalysatoren nach den Vergleichsbeispielen 1 und 3 unterbrochen, nachdem die Konversion am Ende des Versuches (End of Run = EOR) 96,2 bzw. 97,1 % erreicht hatte. Es ist nicht sinnvoll, den Zyklus weiterzuführen, da niedrigere Konversionen technisch uninteressant sind. Die Katalysatoren (auch der erfindungsgemäße Katalysator) wurden nach Beendigung des ersten Zyklus regeneriert, indem zunächst der MeOH-Strom abgestellt wurde. Dann wurde Stickstoff zum Verdrängen des restlichen MeOH eingespeist. Schließlich wurde dem Stickstoff langsam Sauerstoff in allmählich höher werdenden Konzentrationen zugesetzt, um den auf den Katalysatoren abgeschiedenen Kohlenstoff abzubrennen. Die Temperatur der Katalysatoren wurde hierbei immer unter 480°C gehalten. Die Regenerierung der Katalysatoren war beendet, wenn der Sauerstoffgehalt des Stickstoffstromes am Eingang und am Ausgang des Katalysatorbettes der gleiche war.

Zu beachten ist ferner, daß der Katalysator nach Beispiel 1 bei 400°C höhere Konversionswerte zeigt als die Vergleichskatalysatoren, die bei 415°C getestet wurden.

### Anwendungsbeispiel 2

Dieses Beispiel zeigt anhand katalytischer Daten des COD-Verfahrens (Conversion of Olefins to Diesel) in einem isothermen Festbettreaktor, die mit den erfindungsgemäßen Katalysatoren durch die Verwendung des Aluminiumoxid-Bindemittels und die dadurch bedingte Porenradienverteilung erreichbare Verbesserung.

Die Versuche wurden im einzelnen wie folgt durchgeführt:
Das Feed Propen/Buten 1 : 1 wurde bei dem Katalysator nach Vergleichsbeispiel 3 ohne zusätzliches Okten und den in Tabelle V angegebenen Bedingungen in einem isothermen Festbettrohrreaktor über die in der Tabelle V angegebenen Menge an Katalysator geleitet. Die Belastung war mit etwa 0,5 kg Propen/Buten pro kg Katalysator und pro Stunde etwa gleich.

Wenn man davon ausgeht, daß Okten, verglichen mit Propen/Buten ziemlich reaktionsträge ist, so zeigt das Ergebnis, daß der Katalysator nach Beispiel 1 mit dem Aluminiumoxid-Bindemittel deutlich besser bezüglich Selektivität und Umsatz ist, als der Katalysator nach Vergleichsbeispiel 3 mit Bentonit als Bindemittel, obwohl die Primärkristallite der Zeolithkomponente beider Katalysatoren in der gleichen Größenordnung liegen

**Tabelle V**

| Katalysator Bindemittel Primärkrist.(»m) | Vergleichsbeispiel 3 Bentonit 0,3 | Beispiel 1 Aluminiumoxidhydrat 0,3 |
|---|---|---|
| Temperatur (°C) | 301 | 300 |
| Druck (bar) | 50 | 50 |
| Katalysat (g) | 83 | 86,4 |
| Olefin-Einsatz(g/h) | 38 | 45 (Propen/Buten-1=1:1) |
| Okten-Einsatz(g/h) | 0 | 45 |
| Olefin-Konvers. (%) | 40,96 | 67,7 |
| C11+-Bildung (g/g Olefin-feed) | 0,0177 | 0,5437 |
| Zyklus | 2 | 1 |
| Laufzeit im Zyklus(h) | 347 | 451 |

## Patentansprüche

1. Katalysator auf der Basis von kristallinen Alumosilikaten vom Pentasil-Typ mit einem Si/Al-Atomverhältnis von mindestens 10, **dadurch gekennzeichnet,** daß er aus Primärkristalliten mit einem mittleren Durchmesser von mindestens 0,1 »m und höchstens 0,9 »m aufgebaut ist, die zu mindestens 20% zu Agglomeraten von 5 bis 500 »m vereinigt sind, wobei die Primärkristallite bzw. Agglomerate durch feinteiliges Aluminiumoxid, das durch Hydrolyse von aluminiumorganischen Verbindungen erhältlich ist, miteinander verbunden sind, daß seine BET-Oberfläche 300 bis 600 m²/g und sein Porenvolumen (nach der Quecksilberporosimetrie bestimmt) 0,3 bis 0,8 cm²/g beträgt, daß er in der H-Form vorliegt und daß die Menge des feinteiligen Aluminiumoxid-Bindemittels 10 bis 40 Gew.-%, bezogen auf das Gewicht des Endproduktes, beträgt.

2. Katalysator nach Anspruch 1, dadurch gekennzeichnet, daß der mittlere Durchmesser der Primärkristallite im Bereich von 0,1 bis 0,6 »m liegt.

3. Katalysator nach Anspruch 1 oder 2, dadurch gekennzeichnet, daß mindestens 60 % der Poren einen Durchmesser von 14 bis 80 nm besitzen.

4. Katalysator nach einem der Ansprüche 1 bis 3, dadurch gekennzeichnet, daß das feinteilige Aluminiumoxid-Bindemittel im Reaktionsansatz als peptisierbares Aluminiumoxidhydrat vorliegt, wovon mindestens 95 % der Teilchen (auf den mittleren Durchmesser bezogen) ≦ 55 »m sind.

5. Katalysator nach einem der Ansprüche 1 bis 4, dadurch gekennzeichnet, daß das feinteilige Aluminiumoxid-Bindemittel durch Hydrolyse von Aluminiumtrialkylen oder Aluminiumalkoholaten erhältlich ist.

6. Katalysator nach einem der Ansprüche 1 bis 5, dadurch gekennzeichnet, daß er auf folgende Weise erhältlich ist:
(a) in einem wäßrigen Reaktionsansatz, enthaltend eine Siliciumquelle, eine Aluminiumquelle, eine Alkaliquelle und ein Templat, wird bei einer Temperatur von 90°C bis 190°C und gegebenenfalls bei erhöhtem Druck in an sich bekannter Weise ein alkalisches Alumosilicatgel erzeugt und in ein kristallines Alumosilicat umgewandelt, wobei aber die Reaktion abgebrochen wird, wenn die erhaltenen Primärkristallite einen mittleren Durchmesser von mindestens 0,1 »m und höchstens 0,9 »m, vorzugsweise von 0,1 bis 0,6 »m haben;
(b) die Primärkristallite werden aus dem wäßrigen Reaktionsmedium als Voragglomerate abgetrennt, getrocknet und einer Zwischencalcinierung unterzogen;
(c) das Produkt von Stufe (b) wird zum Austausch der Alkaliionen in wäßrigem Medium mit einer protonenhaltigen oder beim Erhitzen Protonen liefernden Substanz umgesetzt, abgetrennt, getrocknet, und erneut einer Zwischencalcinierung unterzogen, worauf eine Agglomeratfraktion von etwa 5 bis 500 »m abgetrennt wird;
(d) die Agglomeratfraktion von Stufe (c) wird mit dem feinteiligen Aluminiumoxidhydrat vermischt;
e) das Produkt von Stufe (d) wird einer Abschlußcalcinierung unterzogen.

7. Katalysator nach einem der Ansprüche 1 bis 6, dadurch gekennzeichnet, daß zur Erzeugung des Alumosilicatgels die Siliciumquelle, die Alkaliquelle, die Aluminiumquelle, das Templat und das Wasser aus den Mutterlaugen vorheriger Synthesen verwendet und durch die für die Synthese des Alumosilicatgels erforderlichen Mengen der genannten Verbindungen ergänzt werden.

8. Katalysator nach einem der Ansprüche 1 bis 7, dadurch gekennzeichnet, daß das Templat Tetrapropylammoniumhydroxid (TPAOH) oder Tetrapropylammoniumbromid (TPABR) ist.

9. Katalysator nach einem der Ansprüche 1 bis 8, dadurch gekennzeichnet, daß das Templat ein Gemisch aus Ammoniak oder einem organischen Amin und einer weiteren organischen Verbindung aus der Gruppe der Alkohole, vorzugsweise Butanol, darstellt.

10. Katalysator nach einem der Ansprüche 1 bis 9, dadurch gekennzeichnet, daß der wäßrige Reaktionsansatz von Stufe (a) einen pH-Wert von 10 bis 13 hat und die Bildung der Alumosilikat-Primärkristallite unter Rühren bei 90 bis 190°C, vorzugsweise bei 90 bis 150°C, erfolgt.

11. Katalysator nach Anspruch 10 dadurch gekennzeichnet, daß die Rührgeschwindigkeit maximal 900 U/min beträgt.

12. Katalysator nach einem der Ansprüche 1 bis 11, dadurch gekennzeichnet, daß die Primärkristallite in Stufe (b) unter Zusatz eines Flockungsmittels aus dem wäßrigen Reaktionsmedium abgetrennt werden.

13. Katalysator nach Anspruch 12, dadurch gekennzeichnet, daß das Flockungsmittel eine kationische organische makromolekulare Verbindung, vorzugsweise ein Copolymer aus Acrylamid und einem kationischen Acrylsäurederivat ist.

14. Katalysator nach einem der Ansprüche 1 bis 13, dadurch gekennzeichnet, daß die Zwischencalcinierung in Stufe (b) in einer inerten Atmosphäre bei etwa 200 bis 350°C, vorzugsweise bei etwa 350°C und anschließend in einer oxidierenden Atmosphäre bei etwa 500 bis 600°C durchgeführt wird, um die gegebenenfalls noch vorhandene Restmenge an Templat abzubrennen.

15. Katalysator nach einem der Ansprüche 1 bis 14, dadurch gekennzeichnet, daß die Zwischencalcinierung in Stufe (c) und die Abschlußcalcinierung in Stufe (e) bei 400 bis 800°C, vorzugsweise bei etwa 540°C, über einen Zeitraum von 5 bis 20 Stunden durchgeführt werden.

16. Verwendung der Katalysatoren nach einem der Ansprüche 1 bis 15 in Methanolumwandlungsprozessen zur Herstellung von Olefinen und Benzin bzw. zur Herstellung von Benzin und/oder Dieselkraftstoffen aus niedermolekularen Olefinen oder niedermolekularen organischen Sauerstoffverbindungen, einschließlich niederen Alkoholen, wie Methanol oder Äthanol und Dimethyläther.

## Claims

1. A catalyst based on crystalline aluminosilicates of the pentasil-type, having an Si/Al atomic ratio of at least 10, characterised in that it is composed of primary crystallites with a mean diameter of at least 0.1 »m and at most 0.9 »m, at least 20 % of which are combined to form agglomerates of 5 to 500 »m, wherein the primary crystallites or agglomerates are bonded together by finely divided alumina which is obtained by hydrolysis of aluminium-organic compounds, in that its BET surface area is 300 to 600 m²/g and its pore volume (determined by mercury porosity) 0.3 to 0.8 cm²/g, in that it is present in H-form and in that the amount of finely divided alumina binding agent is 10 to 40 % by weight, in relation to the weight of the end product.

2. A catalyst according to Claim 1, characterised in that the mean diameter of the primary crystallites is within the range of from 0.1 to 0.6 »m.

3. A catalyst according to Claim 1 or 2, characterised in that at least 60 % of the pores have a diameter of 14 to 80 nm.

4. A catalyst according to any one of Claims 1 to 3, characterised in that the finely divided alumina binding agent is present in the reaction charge in the form of peptisable hydrated alumina, of which at least 95 % of the particles (in relation to the mean diameter) are ≦ 55 »m.

5. A catalyst according to any one of Claims 1 to 4, characterised in that finely divided alumina binding agent is obtained by hydrolysis of aluminium trialkyls or aluminium alkoxides.

6. A catalyst according to any one of Claims 1 to 5, characterised in that it is obtained in the following manner:
(a) in an aqueous reaction charge, containing a source of silicon, a source of aluminium, a source of alkali and a template, at a temperature of 90°C to 190°C and optionally at high pressure, an alkaline aluminosilicate gel is produced in a manner known per se and converted into a crystalline aluminosilicate, however, the reaction being discontinued when the primary crystallites obtained have a mean diameter of at least 0.1 »m and at most 0.9 »m, preferably of 0.1 to 0.6 »m;
(b) the primary crystallites are separated from the aqueous reaction medium as pre-agglomerates, dried and subjected to intermediate calcination;
(c) the product of step (b) is reacted with a substance containing protons or donating protons when heated so as to exchange the alkali ions in an aqueous medium, separated, dried and again subjected to intermediate calcination, whereupon an agglomerate fraction of approximately 5 to 500 »m is separated;
(d) the agglomeration fraction of step (c) is mixed with the finely divided hydrated alumina;
(e) the product of step (d) is subjected to final calcination.

7. A catalyst according to any one of Claims 1 to 6, characterised in that the source of silicon, the source of alkali, the source of aluminium, the template and the water from the mother liquors of previous syntheses are used to produce the aluminosilicate gel and are supplemented by the quantities of said compounds required for the synthesis of the aluminosilicate gel.

8. A catalyst according to any one of Claims 1 to 7, characterised in that the template is tetrapropyl ammonium hydroxide (TPAOH) or tetrapropyl ammonium bromide (TPABR).

9. A catalyst according to any one of Claims 1 to 8, characterised in that the template is a mixture of ammonia or an organic amine and a further organic compound from the group of the alcohols, preferably butanol.

10. A catalyst according to any one of Claims 1 to 9, characterised in that the aqueous reaction charge of step (a) has a pH value of 10 to 13 and the formation of the aluminosilicate-primary crystallites takes place with stirring at 90 to 190°C, preferably at 90 to 150°C.

11. A catalyst according to Claim 10, characterised in that the maximum stirring speed is 900 rpm.

12. A catalyst according to any one of Claims 1 to 11, characterised in that primary crystallites are separated in step (b) with the addition of a flocculant from the aqueous reaction medium.

13. A catalyst according to Claim 12, characterised in that the flocculant is a cationic organic macromolecular compound, preferably a copolymer of acrylamide and a cationic acrylic acid derivative.

14. A catalyst according to any one of Claims 1 to 13, characterised in that the intermediate calcination in step (b) is carried out in an inert atmosphere at approximately 200 to 350°C, preferably at approximately 350°C, and subsequently in an oxidising atmosphere at approximately 500 to 600°C, so as to burn off any residual quantity of template which may still be present.

15. A catalyst according to any one of Claims 1 to 14, characterised in that the intermediate calcination in step (c) and the final calcination in step (e) are carried out at 400 to 800°C, preferably at approximately 540°C, over a period of 5 to 20 hours.

16. Use of the catalysts according to any one of Claims 1 to 15 in methanol conversion processes for the production of olefins and petrol or for the production of petrol and/or diesel fuels from low-molecular olefins or low-molecular organic oxygen compounds, including lower alcohols, such as methanol or ethanol and dimethyl ether.

## Revendications

1. Catalyseur à base d'aluminosilicates cristallins du type pentasil, présentant un rapport atomique Si/Al d'au moins 10, caractérisé en ce qu'il est constitué de cristallites primaires ayant un diamètre moyen d'au moins 0,1 »m et d'au plus 0,9 »m, qui sont réunies pour au moins 20 % en agglomérés de 5 à 500 »m, les particules primaires ou agglomérés étant liés les uns aux autres par de l'oxyde d'aluminium finement divisé, que l'on peut obtenir par hydrolyse de composants organiques de l'aluminium ; en ce que son aire spécifique BET est de 300 à 600 m²/g, avec un volume de pores (déterminé par porosimétrie au mercure) de 0,3 à 0,8 cm²/g ; en ce qu'il se présente sous forme H ; et en ce que la quantité du liant oxyde d'aluminium finement divisé est de 10 à 40 % en poids par rapport au poids du produit fini.

2. Catalyseur selon la revendication 1, caractérisé en ce que le diamètre moyen des cristallites primaires est compris entre 0,1 et 0,6 »m.

3. Catalyseur selon la revendication 1 ou 2, caractérisé en ce que au moins 60 % des pores ont un diamètre de 14 à 80 nm.

4. Catalyseur selon l'une des revendications 1 à 3, caractérisé en ce que le liant oxyde d'aluminium finement divisé est présent, dans la masse réactionnelle, sous forme d'hydrate d'oxyde d'aluminium peptisable, dont au moins 95 % des particules ont un diamètre moyen inférieur ou égal à 55 »m.

5. Catalyseur selon l'une des revendications 1 à 4, caractérisé en ce que le liant oxyde d'aluminium finement divisé est obtenu par hydrolyse de trialkylaluminiums ou d'alcoolates d'aluminium.

6. Catalyseur selon l'une des revendications 1 à 5, caractérisé en ce qu'on l'obtient de la manière suivante :
(a) dans une masse réactionnelle aqueuse, contenant une source de silicium, une source d'aluminium, une source d'alcali et un template, on produit un gel d'aluminosilicate alcalin d'une manière connue en soi, à une température de 90 à 190 °C et éventuellement sous haute pression, et on le convertit en un aluminosilicate cristallin, en interrompant cependant la réaction, lorsque les cristallites primaires obtenues ont un diamètre moyen d'au moins 0,1 »m et d'au plus 0,9 »m, de préférence de 0,1 à 0, 6»m ;
(b) on sépare les cristallites primaires du milieu réactionnel aqueux, sous forme de préagglomérés, on les sèche et on les soumet à une calcination intermédiaire ;
(c) le produit de l'étape (b), pour assurer l'échange des ions de métaux alcalins en milieu aqueux, est mis à réagir avec une substance contenant des protons ou donnant des protons après chauffage, puis on le sépare, on le sèche et on le soumet à une calcination intermédiaire, en séparant ensuite une fraction d'agglomérés d'environ 5 à 500 »m ;
(d) la fraction d'agglomérés de l'étape (c) est ensuite mélangée à l'hydrate d'oxyde d'aluminium finement divisé ;
(e) on soumet le produit de l'étape (d) à une calcination finale.

7. Catalyseur selon l'une des revendications 1 à 6, caractérisé en ce que, pour produire le gel d'aluminosilicate, on utilise la source de silicium, la source d'alcali, la source d'aluminium, le template et l'eau provenant des lessives-mères des synthèses précédentes et on les complète par les quantités des composés mentionnés ci-dessus, nécessaires à la synthèse du gel d'aluminosilicate.

8. Catalyseur selon l'une des revendications 1 à 7, caractérisé en ce que le template est l'hydroxyde de tétrapropylammonium (TPAOH) ou le bromure de tétrapropylammonium (TPABR).

9. Catalyseur selon l'une des revendications 1 à 8, caractérisé en ce que le template est un mélange d'ammoniac ou d'une amine organique et d'un composé organique supplémentaire choisi dans le groupe des alcools, de préférence le butanol.

10. Catalyseur selon l'une des revendications 1 à 9, caractérisé en ce que la masse réactionnelle aqueuse de l'étape (a) a un pH de 10 à 13, et que la formation des cristallites primaires d'aluminosilicate s'effectue sous agitation à un température de 90 à 190 °C et, de préférence, de 90 à 150 °C.

11. Catalyseur selon la revendication 10, caractérisé en ce que la vitesse d'agitation est au maximum de 900 tours/mn.

12. Catalyseur selon l'une des revendications 1 à 11, caractérisé en ce que les cristallites primaires de l'étape (b) sont séparées du milieu réactionnel aqueux par addition d'un floculant.

13. Catalyseur selon la revendication 12, caractérisé en ce que le floculant est un composé macro-moléculaire organique cationique, de préférence un copolymère de l'acrylamide et d'un dérivé cationique de l'acide acrylique.

14. Catalyseur selon l'une des revendications 1 à 13, caractérisé en ce que la calcination intermédiaire de l'étape (b) s'effectue dans une atmosphère inerte à une température d'environ 200 à 350 °C et, de préférence, à environ 350 °C, puis dans une atmosphère oxydante à une température d'environ 500 à 600 °C, pour isoler les quantités résiduelles éventuellement encore présentes du template.

15. Catalyseur selon l'une des revendications 1 à 14, caractérisé en ce que la calcination intermédiaire de l'étape (c) et la calcination finale de l'étape (e) s'effectuent à une température de 400 à 800 °C et, de préférence, d'environ 540 °C, sur un laps de temps de 5 à 20 heures.

16. Utilisation des catalyseurs selon l'une des revendications 1 à 15, dans des procédés de conversion du méthanol destinés à préparer des oléfines et de l'essence, ou à préparer de l'essence et/ou des combustibles diesel, à partir d'oléfines à faibles masses moléculaires ou de composés oxygénés organiques à faibles masse moléculaires, notamment des alcools inférieurs tels que le méthanol ou l'éthanol et l'oxyde de diméthyle.
